# EUROPEAN PATENT APPLICATION

(11) **EP 4 529 898 A1**
(43) Date of publication of application: **02.04.2025**
(21) Application number: 24199855.8
(22) Date of filing: 11.09.2024
(51) Int. Cl.: A61F 2/95, A61M 25/00, A61B 17/12

(54) **SHEATH HUB FOR DELIVERY DEVICES AND METHODS OF MAKING AND USING SAME**

(30) Priority: 26.09.2023 US 202363585269 P
(71) Applicant: St. Jude Medical, Cardiology Division, Inc., St. Paul, MN 55117 (US)
(72) Inventor: Perszyk, Brian Joseph, Shoreview, 55126 (US)
(74) Representative: Gill Jennings & Every LLP

(57) **Abstract**

A delivery device includes a handle having an interior mounting shaft, a catheter sheath extending distally from the handle, and a sheath hub coupled to the catheter sheath, the sheath hub having a body including at least one of a keyed feature for preventing rotation of the sheath hub relative to the handle, and a snap feature for preventing translation of the sheath hub relative to the handle.

## Description

### Cross-Reference to Related Applications

The present application claims priority to U.S. Provisional Serial No. 63/585,269, filed September 26, 2023, the disclosure of which is hereby incorporated by reference in its entirety as if fully set forth herein.

### Background

Catheters are frequently used to assist in the delivery of medical devices into a patient non-invasively. For example, several types of collapsible and expandable medical devices may be delivered to, and implanted within, the heart of a patient using a catheter that is advanced through the vasculature and into the patient's heart without needing to make any incisions in the patient's chest or heart, and without needing to put the patient on cardiopulmonary bypass.

Left atrial appendage ("LAA") occluder devices are one example of collapsible and expandable medical devices that may be delivered to a patient's heart via a catheter that traverses the patient's vasculature. In some examples, a catheter may be advanced through the patient's femoral vein, into the right atrium through the inferior vena cava, across the atrial septum and into the left atrium, with a distal end of the catheter positioned within or adjacent to the LAA. The LAA occluder device may be within the catheter during the advancement of the catheter, or otherwise may be advanced through the catheter after the catheter is already in the desired position. The LAA occluder may be in a collapsed state with a relatively small profile while inside the catheter, and may self-expand into the LAA upon deployment from the distal end of the catheter. One such LAA occluder is the Amplatzer^{™} Amulet^{™} Occluder offered by Abbott Labs. One example of a LAA occluder device is described in U.S. Patent No. 10,201,337, the disclosure of which is hereby incorporated by reference herein.

As explained in greater detail below, although this disclosure generally focuses on a sheath hub in the context of a steerable sheath for delivering a LAA occluder, the disclosure is not so limited, and may apply to various other types of sheaths (including non-steerable sheaths) and various other types of medical devices to be delivered (including other occluder-type devices, such as PFO closure devices, and other devices that are not occluders).

### Brief Summary

In some embodiments, a delivery device includes a handle having an interior mounting shaft, a catheter sheath extending distally from the handle, and a sheath hub coupled to the catheter sheath, the sheath hub having a body including at least one of a keyed feature for preventing rotation of the sheath hub relative to the handle, and a snap feature for preventing translation of the sheath hub relative to the handle.

In some embodiments, a delivery device includes a handle having an interior mounting shaft, a catheter sheath extending distally from the handle, and a sheath hub coupled to the catheter sheath, the sheath hub having a body including a keyed feature for preventing rotation of the sheath hub relative to the handle, and a snap feature for preventing translation of the sheath hub relative to the handle, the keyed feature and the snap feature being at least partially overlapping in an axial direction of the sheath hub.

### Brief Description of the Drawings

Fig. 1 is a schematic view of a steerable sheath according to one aspect of the disclosure.
Fig. 2 is a schematic view of a dilator for use with the steerable sheath of Fig. 1.
Fig. 3A is a schematic cross-sectional view of a sheath hub coupled to an interior mounting shaft of a handle according to the present disclosure.
Fig. 3B is a perspective view of the sheath hub of Fig. 3A.
Fig. 4A is a schematic cross-section of the mounting shaft of the handle according to one embodiment.
Fig. 4B is a schematic cross-section of the keyed feature of a sheath hub within the mounting shaft of Fig. 4A.
Fig. 4C is a schematic side view of the sheath hub of Fig. 3A.
Fig. 4D is a schematic cross-sectional view of a mounting shaft of a handle according to the present disclosure.
Fig. 5A is a perspective view of an occluder in an expanded or deployed condition.
Fig. 5B is a schematic view of the occluder of Fig. 5A in a collapsed or delivery condition.
Fig. 5C is a side view of the occluder of Fig. 5A coupled to a delivery cable.

### Detailed Description

As used herein, the term proximal refers to a position relatively close to a user of a medical device, while the term distal refers to a position relatively far from the user of the medical device, when the medical device is being used in an intended manner. In other words, the leading end of a medical device is positioned distal to the trailing end of the medical device.

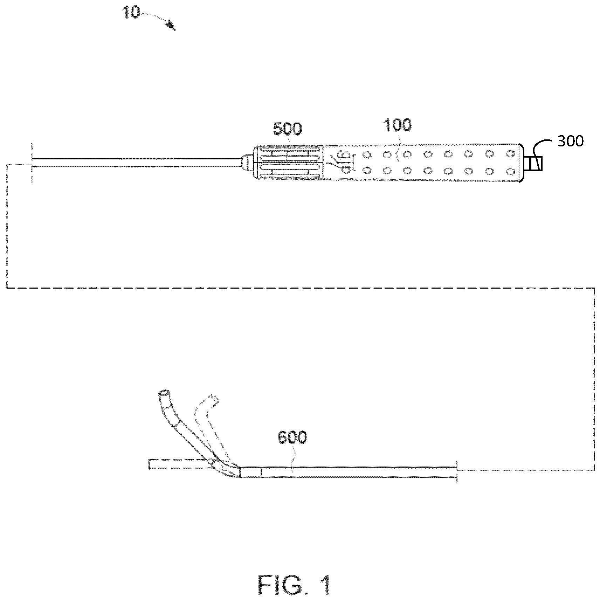

Fig. 1 illustrates a delivery device 10, which in the illustrated embodiment is a steerable sheath, although it should be understood that the inventive concepts disclosed herein may be used in conjunction with other catheter or sheath devices, whether or not steerable. Generally, delivery device 10 includes a handle 100, a sheath hub 300 at a proximal end of the handle 100, a deflection knob 500 at a distal end of the handle 100, and a deflectable sheath 600 extending from a distal end of the deflection knob 500 to a terminal distal end of the delivery device 10. This disclosure focuses on the sheath hub 300, and the remainder of the delivery device 10 is generally described for the purpose of providing a contextual example of the use of sheath hub 300. It should be understood that sheath hub 300 described herein (including the optional variations described therewith) may be applicable to any suitable delivery device. Delivery device 10 is particularly suited for delivery of a collapsible and expandable LAA occluder, but it should be understood that the delivery device 10 may be suited to delivery of other medical devices.

The handle 100 may be a generally cylindrical or otherwise shaped member that the user of the delivery device 10 may grip during use. The handle 100 may be at least partially hollow and house various components therein, and may have one or more internal lumens so that medical devices may be passed through the delivery device 10 from the proximal end to and beyond the terminal distal end of the delivery device 10. The handle 100 may be rotatably coupled to the deflection knob 500, with the deflection knob 500 being rotatable about the central longitudinal axis of the handle 100. The deflection knob 500 may be operably coupled to two pull wires that traverse the deflectable sheath 600 and which are fixed to anchors (or pull rings or similar structures) near the distal tip of the sheath 600. Rotation of the deflection knob 500 in a first rotational direction may deflect the distal tip of the sheath 600 in a first deflection direction, while rotation of the deflection knob 500 in a second opposite rotational direction may deflect the distal tip of the sheath 600 in a second deflection direction opposite the first deflection direction. In one exemplary embodiment, the distal tip of the sheath may have a neutral angled position of about 45 degrees relative to the central longitudinal axis of the delivery device 10, with a maximum deflection (upon rotation of the deflection knob 500 in the first, e.g. clockwise, rotational direction) of about 120 degrees (shown in phantom lines in Fig. 1) relative to the central longitudinal axis of the delivery device 10, and a minimum deflection (upon rotation of the deflection knob 500 in the second, e.g. counterclockwise, rotational direction) of about 0 degrees (shown in phantom lines in Fig. 1) relative to the central longitudinal axis of the delivery device 10. In one example, the proximal end of each pull wire may be coupled to an axially slideable component within handle 100, where rotation of the deflection knob 500 causes the two axially slideable components to slide axially in opposite directions. Suitable pull wire mechanisms are described in greater detail in U.S. Patent No. 7,691,095, the disclosure of which is hereby incorporated by reference herein. The deflection mechanisms and ranges described above are merely exemplary, and as noted above, steering or deflection control may in some embodiments be entirely omitted from delivery device 10.

The catheter 600 may define a lumen therethrough configured to allow other devices to pass through the lumen. The catheter 600 may be formed from any suitable materials and in any suitable configuration. In one example, the catheter 600 includes an innermost liner layer, a torque transfer layer surrounding at least portions of the inner layer, and an outer sheath formed over the torque transfer layer. The wall of the catheter 600 may define lumens as well, for example two lumens spaced about 180 degrees apart, to accommodate the pull wires therethrough. Examples of suitable methods and materials for use in forming the catheter 600 are described in greater detail in U.S. Patent No. 7,914,515, the disclosure of which is hereby incorporated by reference herein. However, it should be understood that sheath hub 300 may be used with any suitable catheter configuration.

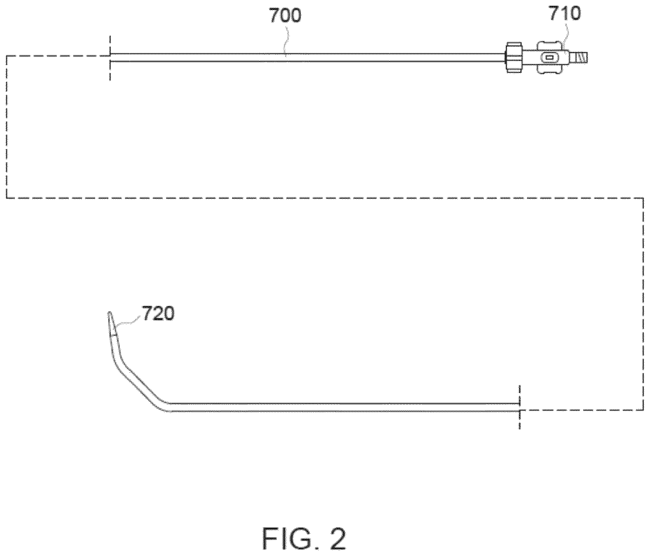

Fig. 2 illustrates a dilator 700 that may be used with delivery device 10. In the illustrated example, dilator 700 is a solid member that includes a connector 710 such as a luer lock at a proximal end thereof, and an atraumatic tip 720 at a distal end thereof. Although referred to as a "solid member," it should be understood that dilator 700 may include a guidewire lumen passing therethrough to allow for the dilator 700 to ride over a guidewire. In other words, dilator 700 may also be thought of as a "substantially solid" or thick-walled device. The distal end portion of the dilator 700 may, in the absence of applied forces, have an angle of about 45 degrees relative to the central longitudinal axis of the dilator 700. In other words, the dilator 700 may include a distal portion that has a neutral angle that is about the same as the neutral angle of the distal tip of the catheter 600, whether that angle is about 45 degrees or another value. The dilator 700 may have an outer diameter that is about equal to (or slightly smaller than) the inner diameter of the catheter 600. As will be described in greater detail below, the dilator 700 may be positioned within and through the catheter 600 during delivery of the delivery device 10 to the desired anatomical location. Then the dilator 700 may be removed to allow for other devices, such as an LAA occluder, to be passed into and through the delivery device 10 for implantation. In some embodiments, the distal portion of the dilator 700 may have curvature, or multiple angles generally similar to that shown in Fig. 2. In an alternate embodiment to the dilator 700 shown in Fig. 2, in other embodiments, the dilator may be completely straight (*e.g.,* without any angles or curvature), or the distal portion may have an entirely straight portion that is at an angle relative to the main body of the dilator 700 (*e.g.,* a single angled portion instead of a multi-angled portion as shown in Fig. 2).

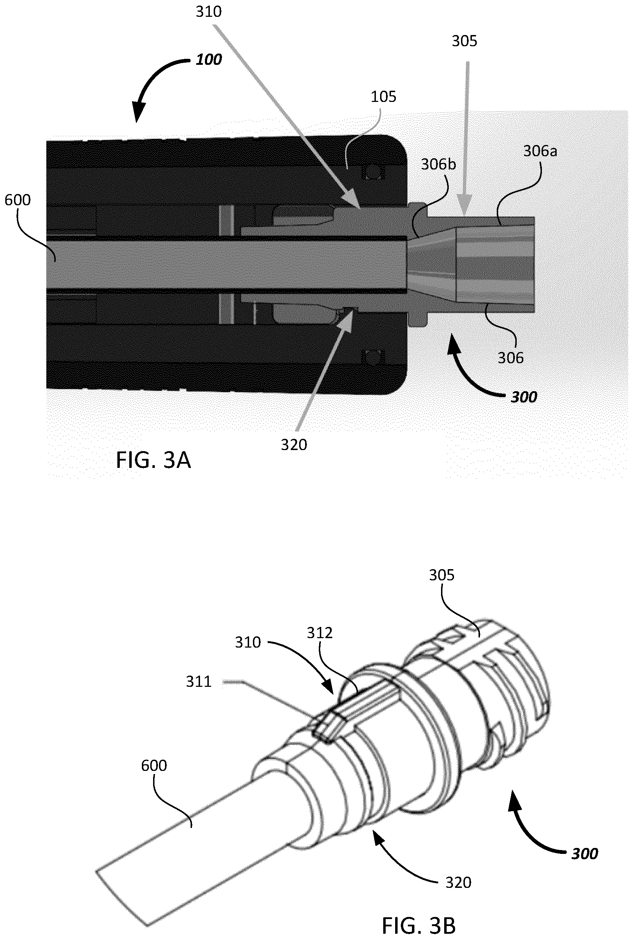

Fig. 3A shows a cross-sectional side view of a sheath hub 300 in an assembled condition with handle 100, and specifically with interior mounting shaft 105 of handle 100. In the assembled condition, sheath hub 300 directly abuts and is coupled to mounting shaft 105 of handle 100, and a proximal end of the catheter 600 is coupled to a distal end of the sheath hub 300. Sheath hub 300 may include a proximal body 305 defining a lumen 306. In some examples, body 305 is molded directly onto catheter 600. Body 305 may be injection molded and made from a suitable polymer (e.g., 63D PEBAX). Body 305 may have a luer-type connection on its proximal end for mating with other instruments (e.g., a loading assembly or other subsystem). In some examples, body 305 including both keyed features and snap features may be unitarily formed. In some examples, lumen 306 has a tapered diameter that decreases from the proximal end of sheath hub 300 to the distal end. In the example shown, lumen 306 includes a multi-stage taper including a first taper 306a and a second taper 306b. Fig. 3A illustrates two additional features of sheath hub 300, each of which will be described in turn. First, sheath hub 300 may include a keyed feature 310 to prevent rotation with respect to handle 100. Second, sheath hub 300 may include a snap-feature 320 to prevent axial or longitudinal movement with respect to handle 100.

As best shown in the perspective view of Fig. 3B, sheath hub 300 may include an axially extending keyed feature 310 in the form of a generally rectangular projection that extends from the outer surface of body 305. In some examples, keyed feature 310 may include a ramp 311 with a gradually increasing height followed by a ridge 312 of constant height. As shown, a keyed feature 310 with a single projection is disposed on body 305, although it will be understood that multiple projections, disposed around the circumference, may be used. In the example shown, the keyed feature 310 includes a single projection disposed at the 12 o'clock position. In some examples, the keyed feature 310 may be disposed in the same plane as the pull wires that traverse the deflectable sheath 600 (e.g., the keyed feature 310, and two pull wires are all disposed within a single plane). Alternatively, keyed feature 310 may be disposed in a different plane than the pull wires that traverse the deflectable sheath 600.

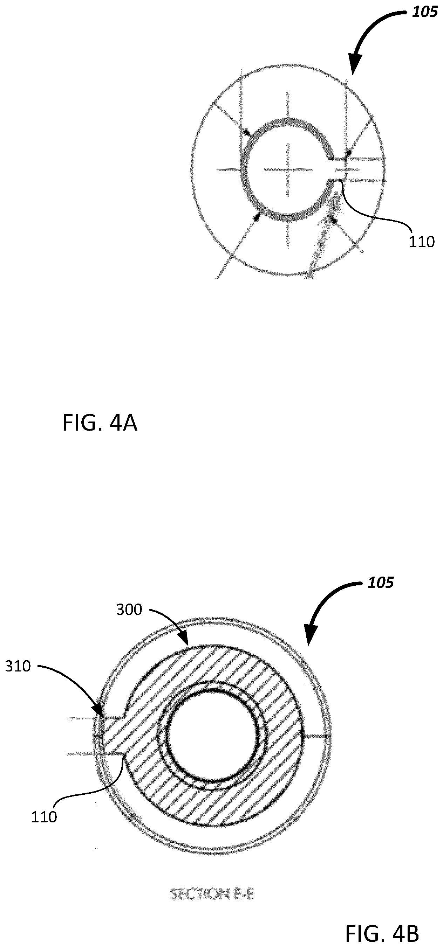

Turning to Figs. 4A-4B, it will be understood that mounting shaft 105 may include a slot 110 sized and spaced to receive keyed feature 310 of sheath hub 300. In some examples, slot 110 may have a same (or similar, or complementary) shape, size, height, and width of keyed feature 310. When keyed feature 310 of sheath hub 300 is properly engaged and/or mated with slot 110 of mounting shaft 105, the sheath hub 300 will be prevented from rotating relative to the mounting shaft 105 and handle 100. This feature may allow the user to safely and securely connect mating loaders, adapters, and/or dilators to the sheath hub 300 without the luer connection spinning in the handle.

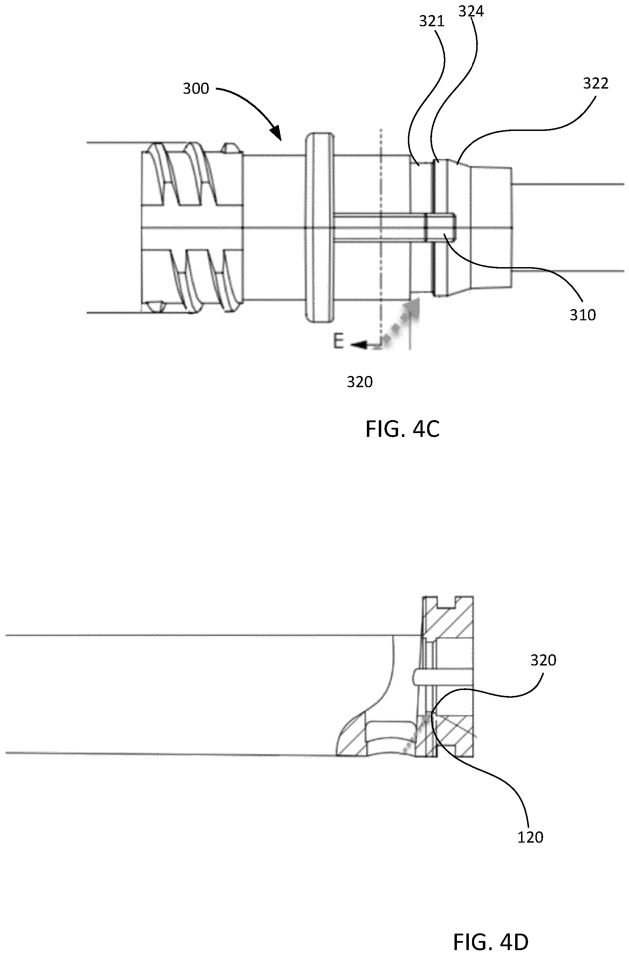

In addition to the keyed feature 310 to prevent rotation, a snap feature 320 (FIG. 4C) may prevent axial movement of the sheath hub 300 with respect to mounting shaft 105. Snap feature 320 may be formed in the shape of a circumferential or annular recess 321 formed in the outer surface of body 305. In some examples, sheath body 305 includes a leading taper 322 that increases in diameter from the proximal end to the distal end, and then forms a ledge 324 with a constant diameter and a sudden drop-off adjacent recess 321. Recess 321 may be continuous or discontinuous. For example, recess 321 may be circumferentially disposed all around body 305 with the exception of the location at which keyed feature 305 is present. That is, the recess 321 may be interrupted by the keyed feature 310.

As shown in Fig. 4D, mounting shaft 105 may have a complementary rim 120 suitable for mating with snap feature 320. Specifically, in assembly, complementary rim 120 may ride onto leading taper 322, over ledge 324 and fall into recess 321 so that sheath hub 300 and mounting shaft 105 are irreversibly coupled and cannot be pulled apart without excessive force. Rim 120 may be continuous or discontinuous. For example, rim 120 may be circumferentially disposed all around mounting shaft 105 with the exception of the location at which keyed feature 310 of sheath hub 300 is present. That is, rim 120 may be interrupted by the keyed feature 310.

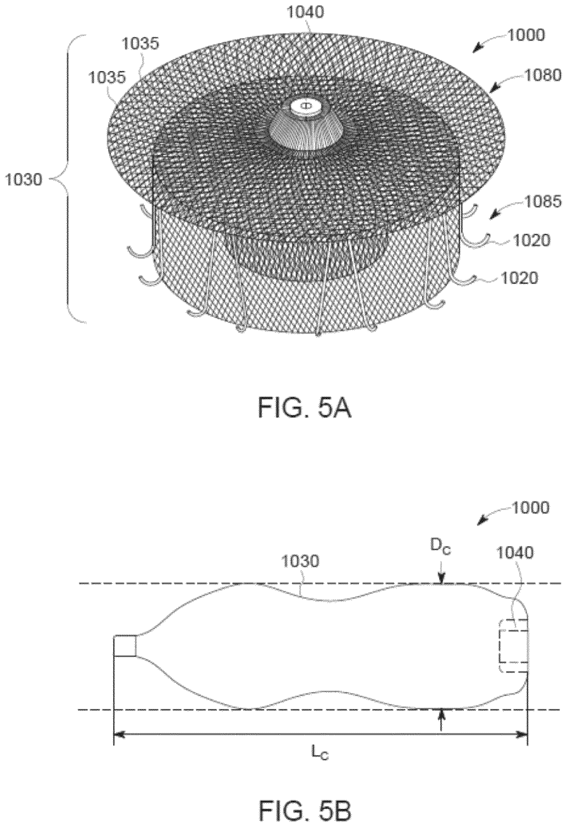

Fig. 5A is a perspective view of an occluder 1000, which may be a LAA occluder. Very generally, occluder 1000 includes an occluding material forming a tubular structure 1030, the occluding material being a braided metal fabric, which may be formed by braiding a plurality of strands 1035, which may be strands of nickel titanium alloy (*e.g.* under the tradename Nitinol), together and shape set (*e.g.* via heat setting) to the shape shown in Fig. 5A. When in the shape-set or expanded condition (*e.g.* in the absence of applied forces), the occluder 1000 may include a generally disk-shaped portion 1080 configured to abut an ostium of the patient's LAA, and a second generally cylindrical portion 1085 configured to be received within the patient's LAA. In some embodiments, the disk-shaped portion 1080 may be coupled to the cylindrical portion 1085 via a small diameter transition portion. The cylindrical portion 1085 may include one or more hooks 1020 configured to engage tissue within the LAA upon deployment. The disk-shaped portion 1080 may include a connector 1040, which may for example include internal threads, for coupling to a delivery cable.

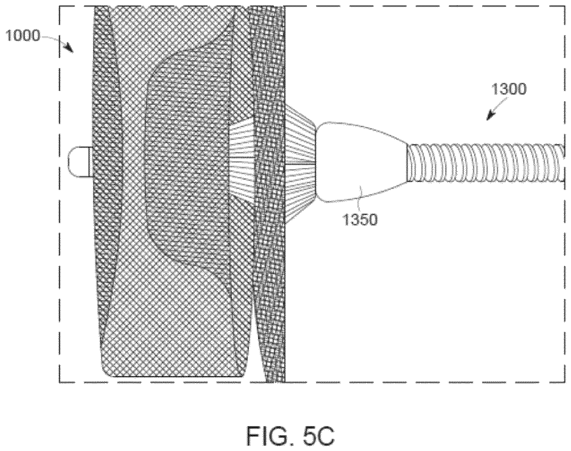

Fig. 5B illustrates occluder 1000 in a collapsed condition, having a collapsed diameter Dc smaller than the diameter of the disk-shaped portion 1080 and the cylindrical portion 1085 when in the expanded condition shown in Fig. 5A, and a collapsed length Lc longer than the length of the occluder in the expanded condition shown in Fig. 5A. Fig. 5C illustrates the delivery cable 1300 coupled to the connector 1040, for example via a threaded hub 1350 of the delivery cable 1300 that has been threaded into the connector 1040.

An exemplary use of the delivery device 10 for delivering occluder 1000 is described below. At the beginning of the procedure (or in preparation thereof), the delivery device 10, including dilator 700, may be removed from sterile packaging. The catheter 600 and the dilator 700 may be wiped with sterile gauze dampened with sterile saline to remove any foreign material that may be on the components. The user may then pass the dilator 700 through the delivery device 10 until the distal end of the dilator 700 passes the distal end of the catheter 600. When the dilator 700 is fully inserted through the delivery device 10, the connector 710 (or a threaded collar associated with the connector 710) may be rotated to couple to sheath hub 300, for example by inner threads of the connector 710 engaging outer threads of the sheath hub 300. Keyed feature(s) 310 and snap feature 320 of sheath hub 300 may prevent accidental detachment throughout the procedure.

The dilator 700 is now coupled to the remaining portions of the delivery device 10 as a single unit. Access may be gained to the patient via any suitable method, and a guidewire may be advanced into the patient's vasculature until it reaches the patient's left atrium, LAA, or pulmonary vein. Prior to or during introduction of the guidewire, a puncture may be made through the patient's atrial septum if the delivery route is, for example, through the patient's femoral vein and to the right atrium via the inferior vena cava, with the septal puncture allowing the guidewire and other components to traverse the atrial septum into the left atrium. With the guidewire in place, the dilator 700 (and the remainder of the delivery device 10 to which it is coupled) may be advanced over the guidewire into the patient until the distal end of the delivery device 10 reaches the left atrium or LAA. With the distal end of the delivery device 10 in the desired location, the connector 710 may be rotated to decouple it from sheath hub 300, and the dilator 700 may be withdrawn from the catheter 600, preferably slowly to prevent any ingress of air. After the dilator 700 is fully removed, the guidewire may next be fully removed from the delivery device 10 and the patient, although the guidewire may instead be simultaneously removed with the dilator 700.

With the distal end of the catheter 600 in the desired position, the occluder 1000 may be introduced into and through the delivery device 10 via a loading tube. Throughout the procedure, the keyed feature 310 and snap feature 320 may secure sheath hub 300 to mounting shaft 105 of handle 100 and prevent unintentional rotation or translation of the two components with respect to one another.

Although the sheath hub described herein may be particularly useful for the LAA occluder 1000 device described herein, it should be understood that this particular use is merely exemplary. For example, the sheath hub described herein may be particularly useful for other occluders formed of braided metal, including septal occluders, patent ductus arteriosus ("PDA") occluder devices, patent foramen ovale ("PFO") closure devices, *etc.* It should further be understood that the sheath hub described herein may work well with other occluders, including those not formed from a braided mesh, or any other medical device, whether an occluder or not and whether formed of braided mesh or not.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A delivery device comprising:
a handle having an interior mounting shaft;
a catheter sheath extending distally from the handle; and
a sheath hub coupled to the catheter sheath, the sheath hub having a body including at least one of a keyed feature for preventing rotation of the sheath hub relative to the handle, and a snap feature for preventing translation of the sheath hub relative to the handle.

2. The delivery device of claim 1, wherein the keyed feature includes a generally rectangular projection extending from an outer surface of the body.

3. The delivery device of claim 1, wherein the keyed feature includes a ramp having a gradually increasing height and a ridge of constant height.

4. The delivery device of claim 1, wherein the keyed feature includes a single projection extending from an outer surface of the body.

5. The delivery device of claim 1, wherein the keyed feature includes multiple projections extending from an outer surface of the body.

6. The delivery device of claim 1, wherein the catheter sheath includes at least one pull wire, and wherein the keyed feature is disposed in a same plane as the at least one pull wire.

7. The delivery device of claim 1, wherein the catheter sheath includes at least one pull wire, and wherein the keyed feature is disposed in a different plane than the at least one pull wire.

8. The delivery device of claim 1, wherein the interior mounting shaft includes a slot configured and arranged to receive the keyed feature.

9. The delivery device of claim 1, wherein the snap feature includes an annular recess formed in an outer surface of the body.

10. The delivery device of claim 9, wherein the snap feature further includes a leading taper disposed adjacent a ledge with a constant diameter and a drop-off adjacent the annular recess.

11. The delivery device of claim 1, wherein the snap feature is continuous about a circumference of the body.

12. The delivery device of claim 1, wherein the snap feature is discontinuous about a circumference of the body.

13. The delivery device of claim 1, wherein the snap feature is interrupted by the keyed feature.

14. The delivery device of claim 1, wherein the interior mounting shaft includes a complementary rim suitable for engaging with the snap feature.

15. The delivery device of claim 1, wherein the sheath hub includes only the keyed feature.

16. The delivery device of claim 1, wherein the sheath hub includes only the snap feature.

17. The delivery device of claim 1, wherein the sheath hub includes both the keyed feature and the snap feature.

18. A delivery device comprising:
a handle having an interior mounting shaft;
a catheter sheath extending distally from the handle; and
a sheath hub coupled to the catheter sheath, the sheath hub having a body including a keyed feature for preventing rotation of the sheath hub relative to the handle, and a snap feature for preventing translation of the sheath hub relative to the handle, the keyed feature and the snap feature being at least partially overlapping in an axial direction of the sheath hub.

19. The delivery device of claim 1, wherein the snap feature comprises a recess circumferentially disposed about the sheath hub at all positions where the keyed feature is not present.

20. The delivery device of claim 1, wherein the body, the snap feature and the keyed feature are unitarily formed.
